Europäisches Patentamt

European Patent Office

Office européen des brevets

(11) Veröffentlichungsnummer: **0 479 217 A1**

## (12) EUROPÄISCHE PATENTANMELDUNG

(21) Anmeldenummer: **91116721.1**

(22) Anmeldetag: **30.09.91**

(30) Priorität: **02.10.90 CH 3154/90**
**27.03.91 CH 961/91**

(43) Veröffentlichungstag der Anmeldung:
**08.04.92 Patentblatt 92/15**

(84) Benannte Vertragsstaaten:
**AT BE CH DE DK ES FR GB GR IT LI LU NL SE**

(51) Int. Cl.5: **A61M 5/32**

(71) Anmelder: **Lamoni, Patrick**
**Via Cattedrale 14**
**CH-6900 Lugano(CH)**
Anmelder: **Dorici, Armando**
**Via dei Bonoli 29**
**CH-6932 Breganzona(CH)**

(72) Erfinder: **Lamoni, Patrick**
**Via Cattedrale 14**
**CH-6900 Lugano(CH)**
Erfinder: **Dorici, Armando**
**Via dei Bonoli 29**
**CH-6932 Breganzona(CH)**

(74) Vertreter: **Blum, Rudolf Emil Ernst et al**
**c/o E. Blum & Co Patentanwälte Vorderberg**
**11**
**CH-8044 Zürich(CH)**

(54) **Einwegspritze mit Sicherheitseinrichtung zur Verhinderung mehrmaligen Gebrauchs.**

㊼ Im Spritzenkörper (1) sind Kapseln (8, 9) vorgesehen, deren Inhalt bei Zerstörung der Kapseln einen expandieren und danach erstarrenden Polyurethanschaum (10) bildet. Am Ende des Kolbenvorlaufs werden die Kapseln (8, 9) zerstört, indem der Kolben (2) eine an der Nadel (6) befestigte Kappe (7) verformt. Der expandierende Schaum (10) zieht die Nadel (6) in den Spritzenkörper (1) zurück. Es ergibt sich eine einfache und billige Möglichkeit, den mehrfachen Gebrauch der Spritze auszuschliessen.

Fig. 3a

EP 0 479 217 A1

Fig. 1

Fig. 3b

Die Erfindung betrifft eine Einwegspritze gemäss Oberbegriff des Patentanspruchs 1. Ferner betrifft die Erfindung eine Sicherheitseinrichtung für eine Einwegspritze gemäss Anspruch 8 bzw. gemäss Anspruch 15.

Einwegspritzen, d.h. steril verpackte Spritzen zum einmaligen Gebrauch, sind heute weit verbreitet. Es ist bekannt, solche Spritzen mit Sicherheitseinrichtungen zu versehen, welche einen unerwünschten mehrmaligen Gebrauch und/oder eine Verletzungsmöglichkeit an der Spritzennadel nach dem Gebrauch der Spritze ausschliessen sollen.

So ist es zum Beispiel aus der EP-A-337 252 bekannt, die Nadel nach Gebrauch an den Spritzenkolben anzukoppeln und die Nadel mittels des Spritzenkolbens in den Spritzenkörper zurückzuziehen. Diese Lösung erfodert einen zusätzlichen, bewusst ausgeführten Arbeitsgang und ergibt daher in der Praxis keine sichere Lösung.

Aus der CH-PS 669 910 ist eine Sicherheitseinrichtung bekannt, bei der einerseits eine mechanische Blockierung den erneuten Gebrauch des Kolbens verhindern soll und andererseits eine Feder das Zurückziehen der Nadel in den Spritzenkörper bewirkt. Diese Sicherheitseinrichtung ist sehr aufwendig.

Der Erfindung liegt deshalb die Aufgabe zugrunde, eine Einwegspritze zu schaffen, deren Sicherheitseinrichtung den erneuten Gebrauch zuverlässig und ohne speziell erforderliche Betätigungsschritte verhindert und welche kostengünstig realisierbar ist.

Dies wird bei einer Einwegspritze der eingangs genannten Art mit den kennzeichnenden Merkmalen des Patentanspruchs 1 erreicht.

Durch die am vorderen Kolbentotpunkt freigesetzte expandierende und erstarrende Substanz kann eine Wiederverwendung der Spritze sicher ausgeschlossen werden. Die durch den in der Regel transparenten Spritzenkörper sichtbare erstarrte Substanz gibt einen optischen Hinweis auf eine nicht mehr verwendbare Spritze.

Vorzugsweise werden der Kolben und die Nadel bei der Expansion einer schaumbildenden Substanz in den Spritzenkörper zurückbewegt und dort bei der Erstarrung der Substanz blockiert. Dadurch wird auf einfache und billige Art sowohl der erneute Gebrauch als auch die Verletzungsgefahr an der Nadel ausgeschlossen.

Ferner liegt der Erfindung die Aufgabe zugrunde, eine universell für Einwegspritzen geeignete Sicherheitseinrichtung zu schaffen. Dies wird dadurch erreicht, dass die Sicherheitseinrichtung eine gegen das Eindringen von Medium abgedichtete, in den Spritzenkörper einsetzbare Kammer umfasst, in welcher, bei einem vorbestimmten Druck des Mediums oder des Kolbens auf mindestens eine Wand der Kammer, die Substanz freisetzbar ist, wobei die Kammer durch die expandierende Substanz zerstörbar ist.

Durch eine separate, gegen das Medium abgedichtete Kammer kann eine Verunreinigung des zu spritzenden Mediums durch die Substanz mit hoher Sicherheit vermieden werden. Die Kammer kann in einen herkömmlich geformten Spritzenkörper eingesetzt werden, um diesen mit der Sicherheitsfunktion auszustatten.

Eine weitere Sicherheitseinrichtung ergibt sich durch eine an den Spritzenkörper ansetzbare Kammer, die als Nadelhalterung dient, und in welcher die Substanz freisetzbar ist. Die chemische Reaktion findet somit vollständig ausserhalb des Spritzenkörpers statt.

Im folgenden werden Ausführungsbeispiele der Erfindung anhand der Zeichnungen näher erläutert. Dabei zeigt

Figur 1 einen Vertikalschnitt durch ein Ausführungsbeispiel der erfindungsgemässen Spritze;

Figur 2 eine Ansicht der Nadel von Figur 1;

Figuren 3 a und 3 b Vertikalschnitte zur Erläuterung der Funktion der Spritze;

Figuren 4 a - 4 f Vertikalschnitte und einen Horizontalschnitt zur Erläuterung der Herstellung der Spritze;

Figur 5 schematisch, teilweise geschnitten, den unteren Bereich einer Einwegspritze mit einer Kammer;

Figuren 6 a, 6 b ein Ausführungsbeispiel der Kammer;

Figuren 7 a, 7 b schematisch, teilweise geschnitten, den unteren Teil einer Einwegspritze zur Darstellung der Funktion;

Figur 8 eine Schnittansicht eines weiteren Ausführungsbeispiels der Kammer;

Figur 9 eine Schnittansicht einer Ausführung der Spritze mit Sicherheitseinrichtung, welche als separate Kammer ausserhalb des Spritzenkörpers ausgeführt ist;

Figur 10 eine Detailansicht der Spritze von Figur 9.

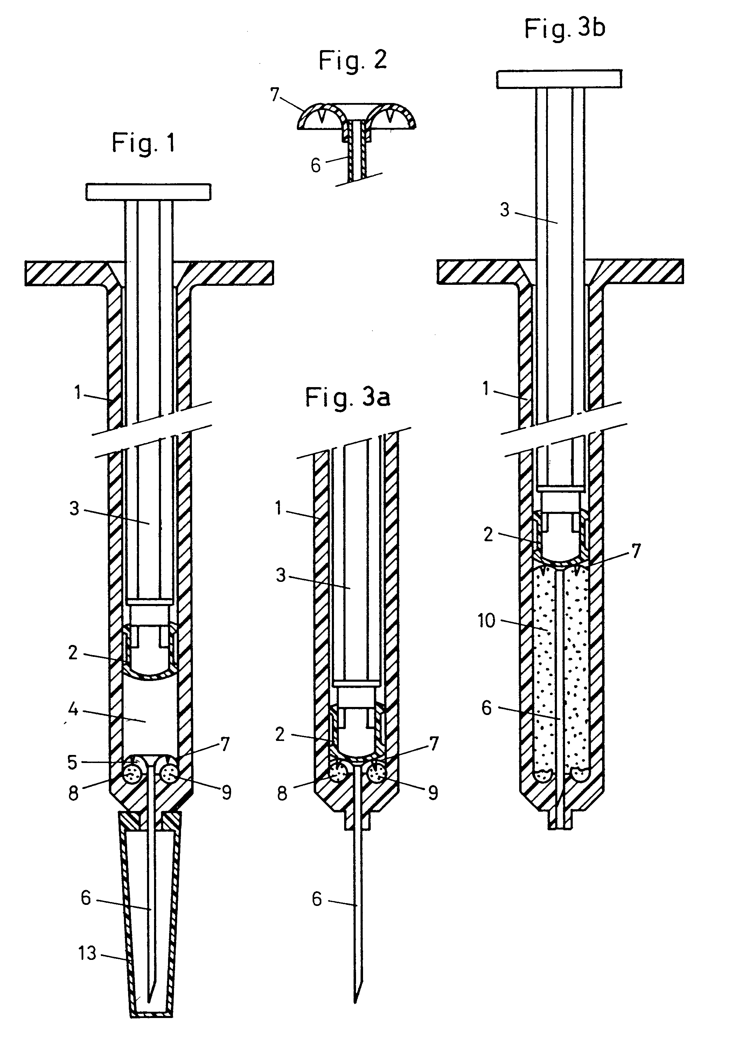

Die Schnittansicht von Figur 1 zeigt einen Spritzenkörper 1, wie an sich bekannt und handelsüblich. In der Regel besteht dieser Spritzenkörper 1 aus transparentem Kunststoff. In den Körper 1 ist ein Kolben 2, vorzugsweise aus halbhartem Gummi, eingeführt, welcher an einem Kolbenschaft 3 befestigt ist. Der Kolbenschaft 3 weist an seinem aus dem Körper 1 ragenden Ende einen Betätigungsflansch auf. Zum Ansaugen der zu injizierenden Flüssigkeit bzw. zur Injektion derselben, wird der Kolben 2 innerhalb des Körpers 1 verschoben, wie dies bei herkömmlichen Spritzen bekannt ist. Die Sicherheitseinrichtung behindert diese Bedienung nicht.

Die Injektionsnadel 6 ist im geschlossenen Ende des Körpers 1 gehaltert. Bei diesem Ausführungsbeispiel weist die Nadel 6 an ihrem spritzenkörperinnenseitigen Ende eine Kappe 7 auf, welche vorzugsweise eine Wölbung aufweist wie in Figur 1 und Figur 2 gezeigt. Die Kappe liegt mit ihrem äusseren Rand an der Innenwandung des Körpers 1 an und unterteilt den Innenraum zwischen dem Kolben 2 und dem geschlossenen Ende des Körpers 1 in einen Raum 4 für die zu injizierende Substanz und einen Raum 5. In dem Raum 5 unterhalb der Kappe 7 sind im gezeigten Beispiel Kapseln 8 und 9 angeordnet, welche die schaumbildenden Stoffe enthalten. Die Kappe 7 besteht vorzugsweise aus relativ weichem Plastik und ist fest am körperinnenseitigen Ende der Nadel 6 fixiert. An der Unterseite der Kappe 7 können dornenförmige Ausformungen vorgesehen sein.

Figur 3 zeigt die Sicherheitsfunktion der Einwegspritze. In Figur 3 a hat der Kolben 2 die zu injizierende Flüssigkeit vollständig aus der Spritze ausgestossen. Die Injektion ist beendet und die Spritzennadel kann aus dem Körper des Patienten herausgezogen werden. Wird nun der Kolben 2 weiter gegen die Kappe 7 gepresst, so verformt sich diese und der Druck des Kolbens wirkt auf die Kapseln 8 und 9, welche so ausgebildet sind, dass sie dabei zerplatzen und ihren Inhalt freigeben. Ist die Unterseite der Kappe 7 mit Dornen versehen, wie gezeigt, so werden die Kapseln 8 und 9 von den Dornen zum Platzen gebracht.

In den Kapseln 8 und 9 ist je ein Stoff A bzw. B eingeschlossen, welche bei Freisetzung im Raum 5 miteinander reagieren und einen Schaum bilden. Vorzugsweise wird ein Polyurethanschaum gebildet, entsprechende Stoffe A und B sind bekannt. Sofern zur Bildung eines bestimmten Schaumes weitere Reaktionspartner oder Treibmittel nötig sind, können natürlich auch weitere Kapseln mit entsprechenden Stoffen vorgesehen werden. Als Kapselmaterial können alle für diesen Zweck bekannten Materialien eingesetzt werden, z.B. Kunststoff, Glas, Gelatine. Der sich nach Freisetzung der Stoffe A und B bildende Polyurethanschaum expandiert, was dazu führt, dass der Schaum die Kappe 7 zusammen mit der Nadel 6 nach oben drückt, in Richtung auf das freie Ende des Spritzenkörpers, wobei der Kolben 2 dieser Bewegung folgt, da er durch die Kappe 7 nach oben gedrückt wird. Figur 3 b zeigt den Zustand am Ende der Expansionsphase des Schaums 10. Das Schlussvolumen des Schaums wird derart gewählt, dass die Nadel 6 vollständig in den Spritzenkörper 1 zurückgezogen wird.

Nach der Schaumbildung beginnt die Aushärtung des Schaums. Die Nadel 6 wird im Spritzenkörper fixiert. Der durch den transparenten Spritzenkörper sichtbare Schaum gibt eine optische Anzeige, dass die Spritze nicht mehr zu verwenden ist.

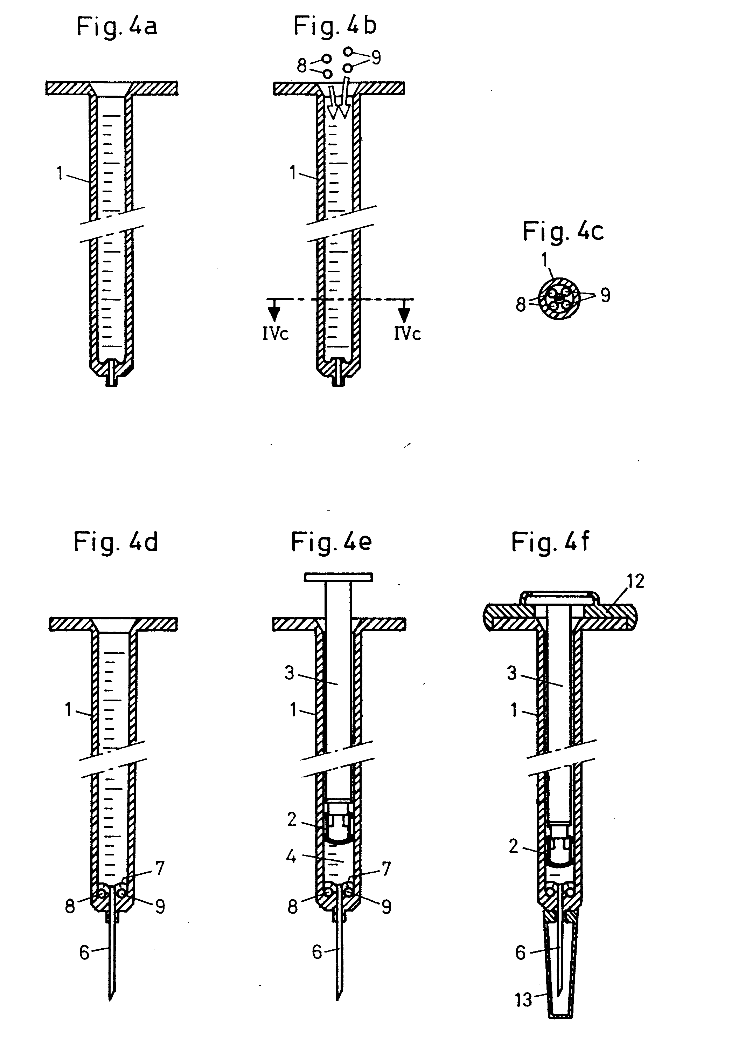

Figuren 4 a bis 4 f zeigen Schritte zur Herstellung der Spritze. Figur 4 a zeigt den Spritzenkörper 1 aus transparentem Kunststoff mit einer Skala für das Volumen (typischerweise von 0 bis 1 ml). Der Körper 1 wird auf bekannte Weise hergestellt, z.B. extrudiert. Figur 4 b zeigt das Einsetzen der Kapseln 8 und 9 mit den schaumbildenden Substanzen A und B. Bei diesem Beispiel sind vier Kapseln, zwei Kapseln 8 und zwei Kapseln 9 vorgesehen. Das nadelseitige Ende des Körpers 1 ist vorzugsweise mit einer umlaufenden, wannenförmigen Vertiefung zur Aufnahme der Kapseln versehen. Es können auch vier halbkugelförmige Vertiefungen vorgesehen sein, um die Lage der Kapseln genauer zu bestimmen. Figur 4 c zeigt einen Horizontalschnitt mit Aufsicht auf die Kapseln.

Figur 4 d zeigt das Einsetzen der Nadel 6, welche bereits mit der Kappe 7 versehen worden ist. Die Nadel 6 wird im Spritzenkörper mittels eines Presssitzes derart gehalten, dass sie für den Einstich bei der Injektion im Körper 1 genügend fixiert ist, indes bei der höheren Rückziehkraft aufgrund der Schaumbildung den Sitz im Körper 1 verlässt und in den Körper 1 hineingezogen werden kann. Die Kappe 7 besteht aus einem halbharten Kunststoffmaterial, welches sich bei Beaufschlagung durch den Kolben 2 verformt, damit die Kapseln zum Platzen gebracht werden können. In der Gegenrichtung - der Krafteinwirkungsrichtung des expandierenden Schaumes - wird durch die Wölbung der Kappe 7 eine genügende Steiffheit der Kappe erzielt, derart, dass über die Kappe, welche vom Schaum beaufschlagt wird, die Nadel 6 aus ihrem Presssitz im Körper 1 lösbar ist. Die Wölbung der Kappe bewirkt weiter, dass der Kappenrand bei der Schaumbildung gegen die Innenwandung des Spritzenkörpers gepresst wird und den expandierenden Raum 5 gegenüber dem Raum 4 abdichtet. Die Befestigung der Kunststoffkappe 7 an der metallenen Nadel 6 muss ebenfalls derart erfolgen, z.B. durch Schweissen oder Kleben, dass die Kappe 7 genügend fest an der Nadel 6 angeordnet ist, damit sich die Kappe 7 nicht von der Nadel 6 löst, wenn der expandierende Schaum auf die Kappe 7 einwirkt, um die Nadel aus dem Presssitz zu lösen.

Figur 4 e zeigt das Einsetzen des Kolbens 2 und des Kolbenschaftes in den Körper 1. Figur 4 f zeigt die zusammengesetze Spritze mit einem Kunststoffsicherungsring 12 für den Kolbenschaft, welcher Ring vor dem Gebrauch der Spritze entfernt werden muss. Ueber der Nadel 6 ist ferner eine Schutzkappe 13 vorgesehen.

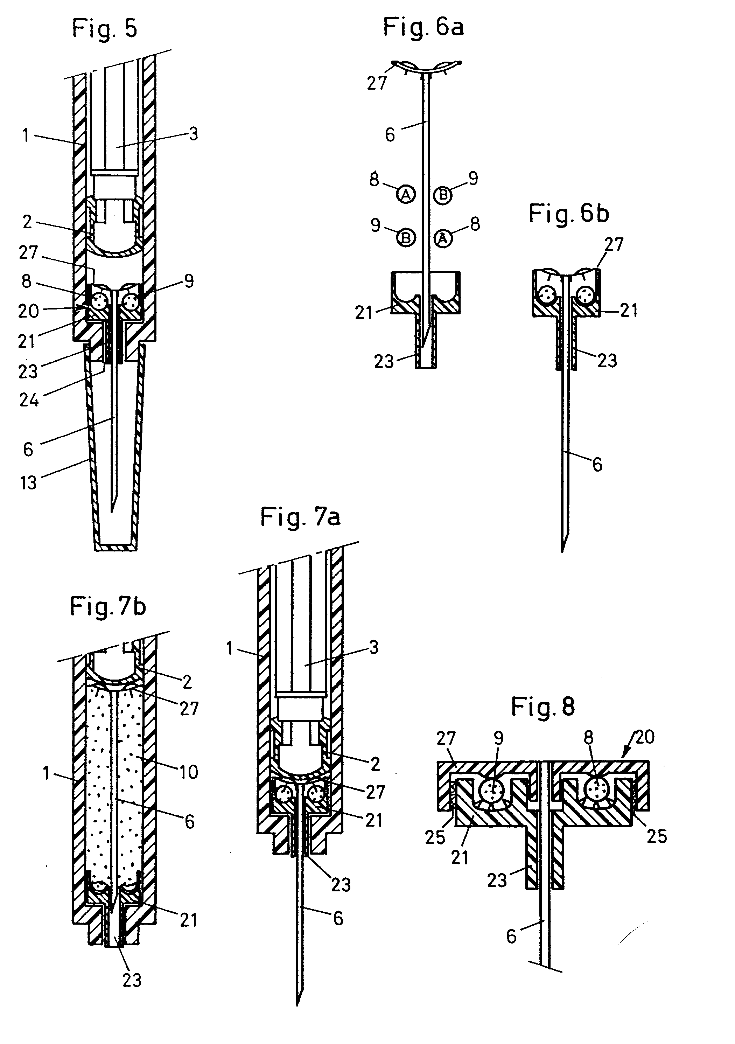

Die in Figur 5 gezeigte Einwegspritze mit der erfindungsgemässen Sicherheitseinrichtung weist eine zusätzliche Kammer auf. In den Spritzenkörper 1 ist die Kammer 20 eingesetzt, in welcher die Kapseln 8, 9 angeordnet sind. Im gezeigten Beispiel besteht die Kammer 20 aus einem Unterteil 21 und einem Deckel 27, welche flüssigkeitsdicht miteinander verbunden sind, z.B. durch Klebung, durch Schweissen oder mittels Feder und Nut sowie einer Dichtpaste. Der Kammerunterteil 21 besteht aus hartem Kunststoff, z.B. aus demselben Material wie der Spritzenkörper. Der Kammerdeckel 27 ist im gezeigten Beispiel ähnlich ausgeführt wie die beschriebene Kappe 7, also aus verformbarem Kunststoff mit Dornen aus härterem Material zur Zerstörung der Kapseln.

Die Kammer 20 ist in ihrem Durchmesser an den Innendurchmesser des Spritzenkörpers angepasst, das heisst, sie füllt das untere Ende des Spritzenkörpers 1 aus. Die Kammer kann im Spritzenkörper durch einen Pressitz gehalten sein oder durch eine Klebverbindung. Im gezeigten Beispiel bildet die Kammer zugleich die Nadelhalterung und der Kammerunterteil 21 ist mit einer Nadelführung 23 versehen, welche in den Spritzenkörper 1 versenkt ist. An der Austrittsstelle 24 können die Nadelführung 23 und der Spritzenkörper 1 miteinander verklebt oder verschweisst sein.

Die Kammer 20 weist ein genau definiertes Volumen auf; um dieses Volumen muss eine auf dem Spritzenkörper 1 angebrachte Skala korrigiert bzw. verschoben werden.

Die Figuren 6 a, 6 b zeigen die Sicherheitseinrichtung für sich alleine, ebenfalls als Nadelhalter ausgeführt. Figur 6 a zeigt die Elemente Kappe 27, Kapseln 8, 9 und Unterteil 21 beim Zusammensetzen, bevor die Kappe und das Unterteil flüssigkeitsdicht verbunden werden. Figur 6 b zeigt die vorbereitete Sicherheitseinrichtung, welche in einen Spritzenkörper eingesetzt werden kann.

Die Figuren 7 a, 7 b zeigen die Funktion der Sicherheitseinrichtung. In Figur 7 a ist gezeigt, wie die Kolben am Ende des Kolbenweges die obere Kammerwand, d.h. die Kappe 27 deformiert, so dass die Kapseln 8, 9 zerstört werden. Figur 7 b zeigt, wie die expandierende Substanz in der Kammer 20 diese zerstört hat. Die obere Kammerwand 27 ist nach oben gedrückt worden und hat die Nadel in den Spritzenkörper gezogen. Der Kolben ist ebenfalls nach oben gedrückt worden und ist nun vom erstarrten Polyurethanschaum blockiert.

Figur 8 zeigt schematisch im Schnitt eine weitere Ausführung der Kammer 20, ebenfalls als Halter für die Nadel 6 ausgebildet, wobei gleiche Bezugsziffern wie vorstehend gleiche Teile bezeichnen. Zwischen dem oberen Teil 27 der Kammer und dem unteren Teil 21 ist eine Dichtung 25 aus Silikonmaterial vorgesehen.

Die Zerstörung der Kapseln 8, 9 kann bei allen Ausführungsbeispielen durch den Druck des Kolbens am Ende des Kolbenweges ausgelöst werden. Alternativ kann die Zerstörung auch durch den Fluiddruck des zu spritzenden Medikamentes ausgelöst werden, also beim Beginn der Injektion, doch muss dann die Reaktion der Stoffe in den Kapseln verzögert erfolgen, damit die Injektion vor der Expansion des Polyurethanschaums abgeschlossen werden kann.

Die Sicherheitseinrichtung in der Form der beschriebenen Kammer bringt auch Vorteile bei der Herstellung, da die Einrichtung separat vom Spritzenkörper, aber zusammen mit der Nadel, montiert werden kann und nachfolgend einfach in einen Spritzenkörper einsetzbar ist.

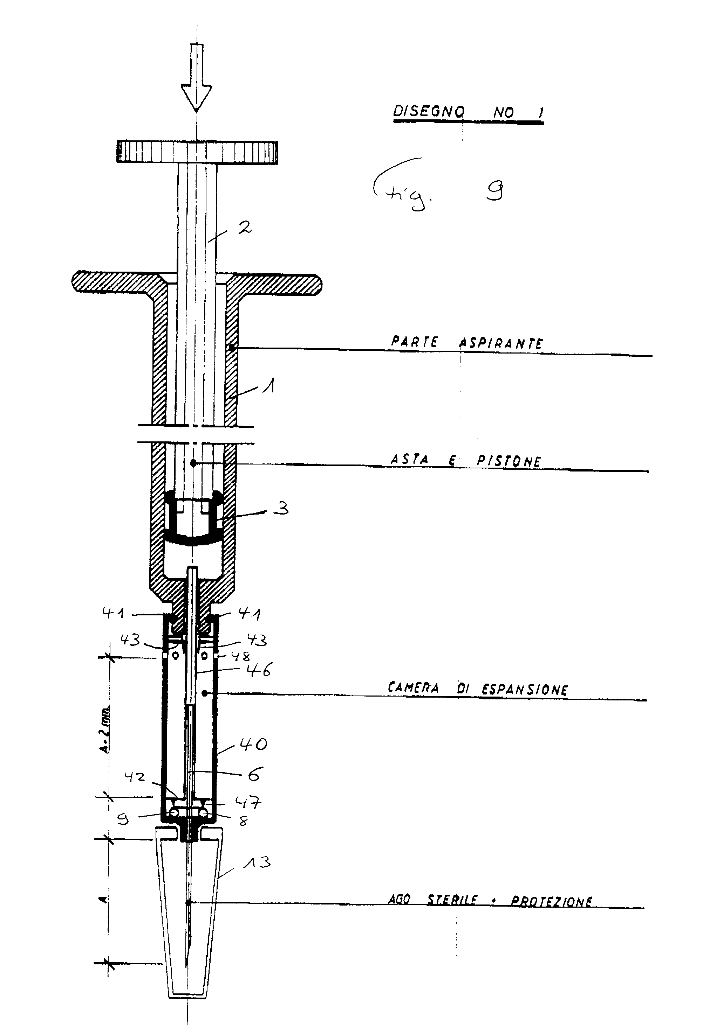

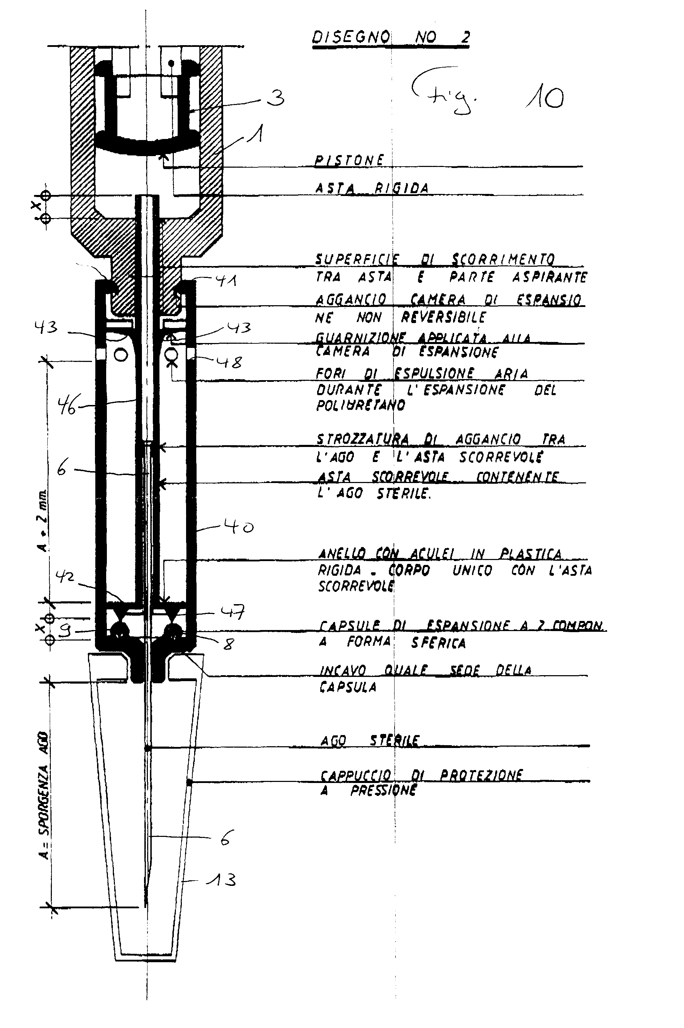

Eine besonders vorteilhafte Ausführung der Sicherheitseinrichtung mit ausserhalb des Spritzenkörpers 1 liegender Kammer 40 ist in den Figuren 9 und 10 gezeigt. Die Funktion ist an sich dieselbe wie vorstehend beschrieben. Die Kammer 40 ist mit einer Schnappbefestigung 41 am Spritzenkörper 1 ansetzbar. Die Kammer haltert die Nadel 6 in einer in der Kammer 40 mittels Führungen 43 längsverschieblich angeordneten Nadelhalterung 46. Diese ist hohl, um das zu spritzende Medium der Nadel 6 zuzuführen. Die Nadelhalterung 46 erstreckt sich mit ihrem oberen Ende in den Spritzenkörper und wird am Ende des Kolbenausstossweges des Kolbens 2 von diesem beaufschlagt. Damit wird die Nadelhalterung 46 nach unten gedrückt. An ihrem unteren Ende weist sie eine Kolbenfläche 42 mit Vorsprüngen 47 auf, welche bei Abwärtsbewegung der Nadelhalterung 46 die in der Kammer 40 befindlichen Kapseln 8,9 zerstören. Die dadurch expandierende Substanz drückt die Kolbenfläche 42 nach oben und somit auch die Nadelhalterung 46 mit der darin befestigten Nadel 6, welche somit in die Kammer 40 zurückgezogen wird. Die sich nach oben bewegende Nadelhalterung 46 drückt auch den Kolben 2 nach oben und blockiert diesen. Entlüftungsöffnungen 48 an der Kammer 40 ermöglichen den Austritt von Luft aus der Kammer.

**Patentansprüche**

1.  Einwegspritze mit einem länglichen Spritzenkörper, an dessen einem Ende die Spritzennadel gehalten ist, einem im Spritzenkörper verschieblich angeordneten, zum Ansaugen und Ausstossen des zu spritzenden Mediums betätigbaren Kolben, und mit einer Sicherheitseinrichtung zur Verhinderung des erneuten Gebrauchs der Spritze, dadurch gekennzeichnet, dass durch die Sicherheitseinrichtung (7, 8, 9) am Ende des Kolbenausstossweges im Spritzenkörper (1) eine expandierende und nach Expansion erstarrende Substanz (10) freisetzbar ist, wodurch mindestens der Kolben (2) in mindestens einer Betätigungsrichtung blockierbar ist.

**2.** Einwegspritze nach Anspruch 1, dadurch gekennzeichnet, dass die expandierende Substanz eine schaumbildende Substanz ist.

**3.** Einwegspritze nach Anspruch 1 oder 2, dadurch gekennzeichnet, dass durch die freigesetzte expandierende Substanz (10) die Nadel (16) vollständig in den Spritzenkörper (1) rückziehbar und darin blockierbar ist.

**4.** Einwegspritze nach Anspruch 3, dadurch gekennzeichnet, dass die Sicherheitseinrichtung eine am spritzenkörperseitigen Ende der Nadel (16) angeordnete, am Spritzenkörper anliegende Kappe (7) umfasst, und dass die Substanz in dem allseits vom Spritzenkörper und der Kappe begrenzten Raum (5) freisetzbar ist.

**5.** Einwegspritze nach Anspruch 4, dadurch gekennzeichnet, dass in dem Raum (5) mindestens zwei Kapseln (8, 9) mit je einem darin enthaltenen Stoff angeordnet sind, welche Stoffe bei Kontakt miteinander die schaumbildende Substanz ergeben, und dass an der Unterseite der Kappe Ausformungen angeordnet sind, durch welche bei Beaufschlagung der Kappe (7) durch den Kolben (2) die Kapseln zur Freisetzung der Stoffe zerstörbar sind.

**6.** Einwegspritze nach Anspruch 5, dadurch gekennzeichnet, dass das nadelseitige Ende des Spritzenkörpers innen mehrere Vertiefungen zur Aufnahme der Kapseln aufweist.

**7.** Einwegspritze nach einem der Ansprüche 2 bis 6, dadurch gekennzeichnet, dass die schaumbildende Substanz ein Polyurethanschaum ist.

**8.** Sicherheitseinrichtung für eine Einwegspritze mit einem länglichen Spritzenkörper, an dessen einem Ende die Spritzennadel gehalten ist, einem im Spritzenkörper verschieblich angeordneten, zum Ansaugen und Ausstossen des zu spritzenden Mediums betätigbaren Kolben, durch welche Sicherheitseinrichtung eine expandierende und nach Expansion erstarrende Substanz freisetzbar ist, wodurch mindestens der Kolben in mindestens einer Betätigungsrichtung blockierbar ist, zur Verhinderung des erneuten Gebrauchs der Spritze, dadurch gekennzeichnet, dass die Sicherheitseinrichtung eine gegen das Eindringen von Medium abgedichtete, in den Spritzenkörper (1) einsetzbare Kammer (20) umfasst, in welcher, bei einem vorbestimmten Druck des Mediums oder des Kolbens, auf mindestens eine Wand (27) der Kammer die Substanz freisetzbar ist, wobei die Kammer durch die expandierende Substanz zerstörbar ist.

**9.** Sicherheitseinrichtung nach Anspruch 8, dadurch gekennzeichnet, dass durch die freigesetzte expandierende Substanz die Nadel vollständig in den Spritzenkörper rückziehbar und darin blockierbar ist.

**10.** Sicherheitseinrichtung nach Anspruch 8 oder 9, dadurch gekennzeichnet, dass in der Kammer mindestens zwei Kapseln mit je einem darin enthaltenen Stoff angeordnet sind, welche Stoffe bei Kontakt miteinander eine expandierende, schaumbildende Substanz ergeben, und dass an mindestens einer Kammerwand gegen die Kapseln gerichtete Ausformungen vorgesehen sind, durch welche die Kapseln zur Freisetzung der Stoffe innerhalb der Kammer zerstörbar sind.

**11.** Sicherheitseinrichtung nach einem der vorangehenden Ansprüche, dadurch gekennzeichnet, dass die Kammer die Halterung für die Spritzennadel bildet.

**12.** Sicherheitseinrichtung nach Anspruch 11, dadurch gekennzeichnet, dass die Spritzennadel durch die Kammer hindurch verläuft und mit ihrem kolbenseitigen Ende an der oberen, kolbenseitigen Kammerwand mündet.

**13.** Sicherheitseinrichtung nach einem der Ansprüche 11 oder 12, dadurch gekennzeichnet, dass die untere Kammerwand mit einer Ausformung (23) zur Führung der Spritzennadel versehen ist.

**14.** Sicherheitseinrichtung nach Anspruch 13, dadurch gekennzeichnet, dass die Ausformung zum Eingriff in eine Ausnehmung des Spritzenkörpers bestimmt ist.

**15.** Sicherheitseinrichtung für eine Einwegspritze mit einem länglichen Spritzenkörper, an dessen einem Ende die Spritzennadel gehalten ist, einem im Spritzenkörper verschieblich angeordneten, zum Ansaugen und Ausstossen des zu spritzenden Mediums betätigbaren Kolben, dadurch gekennzeichnet, dass durch die Sicherheitseinrichtung eine expandierende und nach Expansion erstarrende Substanz freisetzbar ist, wodurch mindestens der Kolben in mindestens einer Betätigungsrichtung blockierbar ist, zur Verhinderung des erneuten Gebrauchs der Spritze, und dass die Sicherheitseinrichtung eine als Nadelhalterung ausgestaltete, an dem einen Ende des Spritzenkörpers befestigbare Kammer aufweist, in welcher das Medium freisetzbar ist.

16. Sicherheitseinrichtung nach Anspruch 15, dadurch gekennzeichnet, dass durch die freigesetzte expandierende Substanz die Nadel vollständig in die Kammer zurückziehbar und darin blockierbar ist.

17. Sicherheitseinrichtung nach Anspruch 15 oder 16, dadurch gekennzeichnet, dass die Nadelhalterung ein in der Kammer verschiebliches Halteelement für die Nadel aufweist, welches in den Spritzenkörper hineinragt und welches vom Kolben beaufschlagbar ist, wobei das Halteelement zur Aufnahme der Nadel und zur Durchleitung des zu spritzenden Mediums ausgestaltet ist.

18. Sicherheitseinrichtung nach Anspruch 17, dadurch gekennzeichnet, dass das Halteelement eine Kolbenfläche aufweist, die mit Ausformungen versehen ist, durch welche bei Beaufschlagung des Halteelementes durch den Kolben (3) in der Kammer angeordnete Kapseln zerstörbar sind, aus denen bei Zerstörung Stoffe austreten, die bei Kontakt miteinander die schaumbildende Substanz geben.

19. Sicherheitseinrichtung nach einem der Ansprüche 15 bis 18, dadurch gekennzeichnet, dass die Kammer am Spritzenkörper mittels einer einrastenden, nicht zerstörungsfrei lösbaren Befestigung gehalten ist.

20. Einwegspritze mit einer Sicherheitseinrichtung nach einem der Ansprüche 8 bis 19.

Fig. 1

Fig. 2

Fig. 3a

Fig. 3b

## Fig. 4a

## Fig. 4b

## Fig. 4c

## Fig. 4d

## Fig. 4e

## Fig. 4f

Fig. 5

Fig. 6a

Fig. 6b

Fig. 7a

Fig. 7b

Fig. 8

DISEGNO NO 1

fig. 9

PARTE ASPIRANTE

ASTA E PISTONE

CAMERA DI ESPANSIONE

AGO STERILE · PROTEZIONE

DISEGNO NO 2

fig. 10

3

1

PISTONE

ASTA RIGIDA

SUPERFICIE DI SCORRIMENTO
TRA ASTA E PARTE ASPIRANTE

AGGANCIO CAMERA DI ESPANSIO
NE NON REVERSIBILE

GUARNIZIONE APPLICATA ALLA
CAMERA DI ESPANSIONE

FORI DI ESPULSIONE ARIA
DURANTE L'ESPANSIONE DEL
POLIURETANO

STROZZATURA DI AGGANCIO TRA
L'AGO E L'ASTA SCORREVOLE

ASTA SCORREVOLE CONTENENTE
L'AGO STERILE.

ANELLO CON ACULEI IN PLASTICA
RIGIDA - CORPO UNICO CON L'ASTA
SCORREVOLE

CAPSULE DI ESPANSIONE A 2 COMPON.
A FORMA SFERICA

INCAVO QUALE SEDE DELLA
CAPSULA

AGO STERILE

CAPPUCCIO DI PROTEZIONE
A PRESSIONE

41

43

43

48

46

6

40

42

47

9

8

6

13

X

A + 2 mm.

A = SPORGENZA AGO

**Europäisches**
**Patentamt**

**EUROPÄISCHER**
**RECHERCHENBERICHT**

Nummer der Anmeldung

**EP 91 11 6721**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P | WO-A-9 104 760 (VAN DEN HAAK)<br>* das ganze Dokument *<br>* insbesondere: * * Seite 2, Zeile 9 - Zeile 14 *<br>* Seite 3, Zeile 6 - Zeile 9 *<br>* Seite 3, Zeile 21 - Zeile 29 *<br>* Seite 3, Zeile 37 - Seite 4, Zeile 4; Ansprüche 2,4 * * | 1-4,8,9 | A 61 M 5/32 |
| Y,P | | 5-7,10-16 | |
| | – – – | | |
| Y | EP-A-0 104 791 (RANGASWAMY)<br>* Seite 4, Zeile 3 - Zeile 5; Abbildung 1 * * | 5-7,10-16 | |
| | – – – – – | | |

RECHERCHIERTE
SACHGEBIETE (Int. Cl.5)

A 61 M

Der vorliegende Recherchenbericht wurde für alle Patentansprüche erstellt

| Recherchenort | Abschlußdatum der Recherche | Prüfer |
|---|---|---|
| Den Haag | 27 November 91 | SEDY, R. |